# EUROPEAN PATENT APPLICATION

(11) **EP 1 552 840 A1**
(43) Date of publication of application: **13.07.2005**
(21) Application number: 04000141.4
(22) Date of filing: 07.01.2004
(51) Int. Cl.: A61K 33/00, A61P 43/00

(54) **Use of a xenon/carbon monoxide mixture for the protection of cells**

(71) Applicant: AGA AB, 181 81 Lidingö (SE)
(72) Inventor: Petzelt, Christian, 12309 Berlin (DE); Jakobsson, Jan, 18254 Djursjolm (SE)
(74) Representative: Schüssler, Andrea, Dr.

(57) **Abstract**

Described is the use of a xenon/carbon monoxide mixture for protecting cells, cell complexes, tissues and/or organs, e.g., from diseased cell turnover, apoptosis, aberrant programming of (unintentional) cell division, necrosis and/or cell death. Preferred clinical indications requiring cell protection or stabilisation of cell cycle/turnover are (a) ischemia reperfusion (e.g., in case of myocardial infarction, stroke and heart surgery), (b) tumour/cancer related disease states and (c) neurodegenerative diseases like all kinds of dementia (e.g., Alzheimer's disease), Parkinson disease and organic psychiatric disease.)

## Description

The present invention relates to the use of a xenon/carbon monoxide mixture for protecting cells, cell complexes, tissues and/or organs, e.g., from damaged cell turnover, apoptosis, aberrant programming of cell division, necrosis and/or cell death.

Xenon (Xe) is an atom (atomic number 54) existing in the ambient atmosphere in a low concentration (0.0000086 % or 0.086 part per million (ppm) or 86 parts per billion (ppb)). When purified it is presented as a gas in normobaric situations. Xe is one of the inert or "Nobel" gases including also argon and krypton. Due to its physiochemical properties xenon gas is heavier than normal air, with a specific gravity or density of 5.887 g/l and its oil/gas partition coefficient is 1.9 and "blood/gas" partition coefficient of about 0.14.

In concentrations of 10-70 vol.% in combination with oxygen xenon exhibits biological effects. A number of studies in humans have looked at the effects of both hyperbaric and normobaric effects of xenon and shown dose dependent analgesic properties similar to those of nitrous oxide and that xenon in higher concentration exhibits anaesthetic properties and creates a drug induced stage of sleep and depression of response to painful stimuli (EP-A-0 864 328; EP-A-0 864 329).

Furthermore, it has been reported that xenon may provide some cell protecting effects against excess release of neurotransmitters (WO-A-00/53192). In vitro studies have shown that xenon decreases the deleterious effects of oxygen depletion. There are also studies documenting positive effects on the cognitive impairment seen after anaesthesia/surgery in experimental animals. In addition, it has been reported that xenon administration during early reperfusion reduces infarct size after regional ischemia in the rabbit heart (Preckel et al., Anesthesia and Analgesia, Dec. 2000, 91(6), 1327-1332).

The exact mechanism of action for the effects of xenon is not entirely clear. During recent years a number of studies have elucidated that xenon exhibits effects on the NMDA transmission and xenon has been used as NMDA receptor antagonist (US-B-6,274,633). Whether these effects mostly relate to a decreased release of NMDA or a blocking effect on the NMDA receptor on the postsynaptic membrane is hard to tell. These effects have been claimed to be dose dependent and high concentrations, more than 50 vol.% have been suggested to be required for clinical effects. These high concentrations are associated with profound effects on wakefulness. It is rather clear that humans breathing more than 50 vol.% will enter a light stage of anaesthesia.

However, these anaesthetic side effects of xenon based on the requirement of quite high concentrations of xenon render its therapeutic use less than perfect.

Therefore, it is the object of the present invention to improve and extend the therapeutic applications of xenon.

According to the invention this is achieved by the subject matter defined in claim 1. Further advantageous embodiments and aspects of the present invention follow from the dependent claims, the description and the drawings.

During the experiments leading to the present invention it was unexpectedly found that the therapeutic effects of xenon can be improved and extended by using xenon in combination with carbon monoxide.

Carbon monoxide is a well known intoxicant produced during combustion of organic fuels. Carbon monoxide intoxication is one of most common intoxications seen. It is known since several years that CO is formed during the turnover of haemoglobin and that carboxyhaemoglobin (COHB) is a sensitive measure of the haemoglobin breakdown. At least two iso-enzymes have been identified, heme-oxygenase II (HO-II) is a constitutive enzyme and an inducible form is HO-I. HO-I has been shown to be involved in the modulation of the cascade of effects caused by a number of stressful situations such as transplantation, ischemia reperfusion and/or inflammation. Resent studies have shown that exogenous inhaled ppm concentrations of CO for shorter periods of time (60 to 120 minutes) also exhibit a variety of physiological effects similar to those of HO-I. Inhalation of carbon monoxide is, however, associated to side effects related to its high affinity for heme, thereby blocking physiological effects of heme-containing proteins, e.g. reducing haemoglobin oxygen carrying capacity causing CO related hypoxaemia. CO also binds to intracellular heme-containing enzymes blocking/interfering with cytochromoxidase. Furthermore, it has been reported that carbon monoxide improves outcomes in tissue transplants and organ transplants and suppresses apoptosis (WO-A-03/000114). In addition, it has been shown that carbon monoxide may be used for treating inflammation of the upper airways and bronchi (WO-A-02/09731). A further use of carbon monoxide is as a biomarker and therapeutic agent of several heart, lung, liver, spleen, brain, skin and kidney diseases (WO-A-03/094932). WO-A-98/13058 provides a method for treating an ischemic disorder in a subject which comprises administering to the subject carbon monoxide gas in a sufficient amount over a sufficient period of time.

In the present invention, unexpectedly the mixture of CO and xenon of the present invention shows synergistic effects on cell protection.

Thus, said mixture is useful for protecting cells, cell complexes, tissues and/or organs, e.g., from damaged cell turnover, apoptosis, aberrant programming of cell division, necrosis and/or cell death. Preferred clinical indications requiring cell protection, cell stabilisation of cell cycle/turnover are (a) ischemia reperfusion (e.g., in case of myocardial infarction, stroke and heart surgery), (b) tumour/cancer related disease states and (c) neurodegenerative diseases like all kinds of dementia (e.g., Alzheimer's disease, Parkinson disease) and organic psychiatric disease. The combination of gaseous CO and xenon can create clinically relevant effects in concentrations avoiding potential undesirable effects/side effects, e.g., CO anaemia/hypoxaemia and xenon related sedation/anaesthesia while still maintaining clinically relevant cell-protective properties.

Thus, the present invention generally relates to the use of a xenon/carbon monoxide gas mixture or a composition containing said mixture for the preparation of a pharmaceutical composition for protecting cells, cell complexes, tissues and/or organs from damaged cell turnover, apoptosis, aberrant programming of cell division, necrosis and/or cell death.

If "xenon/CO" or "xenon/carbon monoxide mixture" is mentioned this also includes xenon/CO gas mixtures and it is not intended to restrict the invention to a pure mixture of xenon and carbon monoxide. These additional gases may be oxygen, nitrogen, (ambient) air, etc. To the above mentioned xenon/CO and xenon/CO gas mixtures also other nobel gases, like helium, may be added. Since helium is a molecule of small size it may function as carrier for the more voluminous xenon. Furthermore, further gases having medical effects may be added, e.g. NO or CO₂. In addition, depending on the disease to be treated other medicaments which are preferably inhalable may be added, e.g. cortisones, antibiotics etc.

The term "damaged cell turnover" as used herein refers to a situation where external stress factors induce transient or permanent cell damage leading to cellular death.

The term "aberrant programming of cell division" as used herein refers to the fact that often existing tumor cells in the organism reside in dormant form. Upon an external stimulus they are activated, enter the cell cycle and become aggressive.

A preferred use of the xenon/carbon monoxide mixture of the present invention or the composition containing said mixture is to protect cells exposed to ischemia or hypoxia, particularly for ischemia reperfusion. Preferred cells are cells of the heart, brain, kidney or peripheral tissue (POAD).

The ischemia/reperfusion (I/R) injury is a major problem, e.g., in liver transplantation or hepatic resection with ischemic procedure, in addition to hepatocyte hypoxemic damage. A burst in production of hydrogen peroxide (H₂O₂) occurring during the reperfusion phase may have a detrimental effect on the organ being reperfused. Immediately after reperfusion, hepatocytes and Kupffer cells generate reactive oxygen species (ROS), including H₂O₂. Subsequently, activated neutrophils, which infiltrate the liver tissue, also produce ROS during the latter phase of reperfusion. H₂O₂-treated cells lead to apoptotic and necrotic death.

A further preferred use of the xenon/carbon monoxide mixture of the present invention or the composition containing said mixture is the protection of the quiescent state of potential tumor cells in organs or tissues.

Furthermore, the xenon/carbon monoxide mixture of the present invention or the composition containing said mixture is useful for protection of neural tissue in a potentially damaging situation, i.e., the stopping of the programmed cell death induced by external or internal factors. Preferred examples of such diseased states are dementia diseases like Alzheimer's disease, Parkinson disease or an organic psychosis.

Furthermore, it is possible to use the xenon/CO mixture as prophylactic means to pretreat patients at risk.

All concentrations below are volume based.

For most indications it is desirable to avoid increasing sedative effects of xenon in concentrations above 25 - 30 vol.% and potentially deleterious effects of CO when COHb is higher than 8 - 10 %. Thus, the following amounts are preferred:
Xenon 1 - 25 vol.%, preferred 5 - 20 vol.%, particularly preferred 10 - 15 vol.%
CO 10 - 100 ppm (0.001 - 0.01 vol.%).

However, for some indications (e.g. acute brain ischemia) it is desirable to mix xenon and CO in higher concentrations to use the normally undesired xenon sedative effect. This mixture is suitable for short time uses and enhances the known effects of xenon. For these specific indications preferred concentrations are:
Xenon 25 - 75 vol.%, preferred 30 - 60 vol.%, particularly preferred 40-50 vol.%
CO 10 - 500 ppm, preferred 100 - 400 ppm, particularly preferred 200 - 300 ppm.

It should be noted that the delivered "dose" most certainly is related to:
1. concentration in inhaled gas
2. duration of administration
3. mode of ventilation

The administration of the xenon/CO or xenon/CO gas mixture is preferably via inhalation to ensure a sufficient xenon concentration in the blood. In the case of acute life threatening states, respiration can advantageously be carried out with a xenon/CO:oxygen mixture of 90:10% by volume, preferably 80:20% by volume, most preferably 75-70:25-30% by volume, over several hours to one day. As compared thereto, the intermittent respiration of a xenon/CO-air mixture to which less xenon/CO has been added, e.g., 5 to 30% by volume of xenon/CO, preferably 10 to 20% by volume of xenon/CO, can be considered in chronic progression of a disease.

Compressed or pressurized gas useful for the present invention can be obtained from any commercial source, and in any type of vessel appropriate for storing compressed gas. For example, compressed or pressurized gases can be obtained from any source that supplies compressed gases, such as xenon, carbon monoxide, oxygen etc. for medical use. The pressurized gases can be provided such that all gases of the desired final composition are mixed in the same vessel. Optionally, the present invention can be performed by using multiple vessels containing individual gases.

The protection of cells etc. for the clinical indications listed above and below, respectively, can be carried out according to the present invention on the basis of a simple inhalation therapy. The uptake of xenon via the respiratory system and the transport into the brain are already proven by the use of xenon as an anaesthetic agent. It can also be assumed that the use of xenon has no damaging effect on an organism since many corresponding experiences could be made already by its use as anaesthetic agent. The xenon/CO mixture can be applied by various techniques which can be chosen depending on the particular use. For example, an inhaling apparatus can be used in the clinics that is already used for anaesthesia by inhalation and in which pressure vessels containing prefabricated xenon-oxygen mixtures and CO can be connected to the corresponding inlets of the apparatus. The respiration is then carried out according to a procedure common for such apparatus.

If a cardio-pulmonary bypass machine or another artificial breathing apparatus are used, xenon/CO can be added directly in the machine and requires no further apparatus. On an ambulant basis, e.g., in case of an emergency, it is even possible to use simpler inhalators which mix xenon/CO with the ambient air during the process of inhalation. In this connection, it is also possible to adapt the xenon/CO concentration and the timing of xenon/CO application in a simple manner to the therapeutic requirements. For example, it might be advantageous to use mixtures of xenon/CO with other gases harmless for humans, e.g., oxygen, nitrogen, air etc.

The use of Xe/CO in combination with "fraction of inspired oxygen" (FiO₂) of at least ambient air, that is 21 vol.%., or eventually in combination with an "increased FiO2" supplementary oxygen is preferred.

As an alternative, xenon/CO can be mixed with ambient air instead of oxygen in the mobile use, which due to the smaller size of the required pressure vessels increases the mobility of the apparatus. For example, it is possible to use an inhalator which supplies xenon and CO from pressure vessels and is accommodated in a support together with the latter to a mixing chamber. On one side, this mixing chamber contains a mouthpiece for inhaling the xenon and CO and on the other side on which the xenon/CO is supplied to the mixing chamber it has at least one additional check valve which enables the inlet of ambient air. The xenon and CO pressure containers can be equipped with a pressure reducing valve, e.g., a valve which reduces the amount of xenon gas supplied to a suitable value. When the patient breathes in, he sucks in air from air valves. In the mixing chamber, this air is mixed with the supplied xenon and CO to the desired ratio and then inhaled by the patient. An advantageous inhalator suitable for mobile use and serving for inhaling xenon and its mixtures is, e.g., described in EP-B1-0 560 928.

For self-medication a mouthpiece can be connected directly to the xenon and CO pressure vessels. During inhalation the patient opens the pressure valve and inhales xenon and CO simultaneously with the air from the environment. When the patient breathes out, she/he releases the valve, so that no more xenon and CO reach the mouthpiece. In this way, at least a rough regulation of the amount of inhaled xenon and CO is possible.

Alternatively, e.g., for preserving tissue or organs to be surgically treated, the xenon/CO mixture can be administered as a xenon/CO-saturated solution. A buffered salt solution (Petzelt et al., Life Sci. 72, 1909-1918 (2003)) is exposed to 100% xenon/CO, or alternatively 80% xenon/CO:20% oxygen air-tight plastic bag and mixed for one hour on a shaker. The gas atmosphere is changed at least one time and the mixing procedure is repeated. Then a complete saturation of the buffer with the gas (mixture) is achieved. This solution is particularly useful for preventing aberrant programming of tumor cells in given surgical situations.

The following examples illustrate the invention.

### Example 1: Treatment of neurons undergoing severe hypoxia; measurement of long-term survival

Embryonic rat cortical neurons can survive in a differentiated state *in vitro* for several weeks preserving their neuronal integrity. If they undergo an episode of severe hypoxia by maintaining them for 2 hours in an atmosphere containing nitrogen, a part of them will die immediately, most of them will perish after hours or days following the hypoxia-induced cell death. If these cells are maintained after the initial hypoxic insult of 2 hours for another 2 hours in a buffer solution (Petzelt, 2003) containing 0.001% CO, their survival rate is improved although still more than 50% will ultimately die. If the buffer solution is saturated with xenon and the cells are treated with it after the initial 2 hour hypoxic insult, more than 70% of the neurons will survive. Finally, a treatment of the cells with a combination of the two, i.e., treating them for 2 hours after the first hypoxic insult with a buffer solution saturated in 99.999% xenon, 0.001% CO, almost all neurons survive the hypoxic treatment.

### Example 2: Wounding of a quiescent culture of various tumor cells, e.g. human melanoma, human uterus epithelioma, and subsequent analysis of cell proliferation rate

Although it is widely accepted that one characteristic of tumor cells is their ability to continue proliferation in spite of being in a confluent state (compared to normal cells which stop proliferating upon reaching confluency), such statement cannot be generalized. Many tumor cells in the organism (as well as in vitro) become quiescent and can remain as such for a considerable period of time. In contrast to normal cells, however, often an external stimulus suffices to induce them to reenter the cell cycle and continue their proliferation, often even showing an accelerated proliferation rate. Such stimulus can be an internal wounding as it may occur during surgery. It can be imitated by artificial wounding of a quiescent cell culture *in vitro.*

Human primary melanoma cells derived from individual biopsies are grown to confluency as known in the art. Half of the medium will be replaced by fresh medium in order to assure that quiescence has not been reached by the exhaustion of the medium. Then the cells are washed and maintained for 2 hours, either in buffer saturated with 100% xenon, or in buffer saturated with 0.001 % CO or in buffer saturated with 99.999% xenon, 0.001% CO. Thereafter, using a fine needle, with a broad scratch about 10% of the cells are taken off the culture and the medium replaced by normal medium. During the following three days the proliferation kinetics of the culture is analyzed (by determination of the mitotic index and videomicroscopy).

Results: As expected, the wounding stimulus causes an immediate resumption of proliferation activities within an untreated culture. It becomes also evident that at the site where the wounding had been afflicted, proliferation continues beyond the newly reached confluent state, cells start to grow in three dimensions. If cells are treated before the wounding with buffer containing 0.001% CO, a small but significant reduction in their subsequently induced proliferation rate is observed. A considerable slowdown occurs when cells are incubated in buffer saturated with 100% xenon. After the wounding it takes two times longer to fill the wound than in an untreated culture. However, still an - albeit much reduced - overshooting of the proliferation activity is found, a small percentage of cells grow in three dimension at the site of the original wound. That latter effect is almost completely suppressed if the culture is pretreated before the wounding with buffer saturated with 99.999% xenon, 0.001% CO. The cells repopulate the wounding area, much slower than untreated controls, and stop then in their majority when confluency is reached.

These experiments may be taken as evidence that the presence of a buffer solution during surgery containing 99.999% xenon, 0.001% CO may prevent activation of potentially aggressive tumor cells.

## Claims

1. Use of a xenon/carbon monoxide mixture or a composition containing said mixture for the preparation of a pharmaceutical composition for protecting cells, cell complexes, tissues and/or organs from diseased cell turnover, apoptosis, unintentional cell division, necrosis and/or cell death.

2. Use according to claim 1 for protecting cells exposed to ischemia or hypoxia.

3. Use according to claim 2 for ischemia reperfusion.

4. Use according to claim 2, wherein said cells are cells of the heart, brain, kidney or peripheral tissue (POAD).

5. Use according to claim 1, wherein said cells are cells undergoing aberrant programming into actively dividing tumour cells.

6. Use according to claim 1, wherein said tissue is neural tissue in a damaged state.

7. Use according to claim 6, wherein said diseased state is caused by a dementia disease, Parkinson disease or an organic psychosis.

8. Use according to any one of claims 1 to 7 wherein the concentrationas of Xe and CO are:
Xenon 1 - 25 vol.%
CO 10 - 100 ppm.

9. Use according to any of claims 1 to 7 wherein the concentrations of Xe and CO are:
Xenon 25 - 75 vol.%
CO 100 - 500 ppm.

10. Use according to any one of claims 1 to 9, wherein said pharmaceutical preparation contains 5 to 90% by volume of said xenon/carbon monoxide mixture.

11. Use according to claim 10, wherein said pharmaceutical preparation contains 5 to 30% by volume of said xenon/carbon monoxide mixture.

12. Use according to any one of claims 1 to 11, wherein said pharmaceutical preparation additionally contains oxygen, nitrogen and/or air.

13. Use according to any of claims 1 to 12, wherein said pharmaceutical preparation additionally contains helium, NO, CO₂, other gaseous compounds and/or inhalable medicaments.

14. Use according to claim 10 wherein said pharmaceutical preparation has a ratio of said xenon/carbon monoxide mixture to oxygen of 80 to 20% by volume.

15. Method of preparing a pharmaceutical preparation by mixing xenon and carbon monoxide with another gas harmless to humans.

16. The method of claim 15, wherein xenon and carbon monoxide are mixed with an oxygen-containing gas.
